# EUROPEAN PATENT SPECIFICATION

(11) **EP 2 664 690 B1**
(45) Date of publication and mention of the grant of the patent: **16.09.2015**
(21) Application number: 12250163.8
(22) Date of filing: 19.10.2012
(51) Int. Cl.: C23C 14/02, C23C 14/06, C23C 14/34, C23C 14/35, C23C 14/50, C23C 28/00, H01J 37/32, H01J 37/34, A61L 27/30

(54) **A magnetron sputtering coating device and the preparation method of a nano-multilayer film**
Magnetron-Sputter-Beschichtungsvorrichtung und Herstellungsverfahren für Nano-Mehrschichtfolie
Dispositif de revêtement par pulvérisation au magnétron et procédé de préparation d'un film nano-multicouche

(30) Priority: 15.05.2012 CN 201210151152; 15.05.2012 CN 201220218296 U
(43) Date of publication of application: 20.11.2013
(60) Divisional application: 15000965.2
(73) Proprietor: ZhongAo HuiCheng Technology Co. Ltd., Beijing 100076 (CN)
(72) Inventor: Jin, Gong, Beijing 100085 (CN); Tu, Jiangping, Beijing 100085 (CN); Li, Lingling, Beijing 100085 (CN); Wang, Gang, Beijing 100085 (CN); Wang, Meina, Beijing 100085 (CN)
(74) Representative: Atkinson, Ralph

(56) References cited:
- CN-A- 102 247 620
- DE-A1-102008 062 332
- US-A1- 2001 031 346
- US-A1- 2007 111 003
- GIOTI M ET AL: "Stress relaxation and stability in thick amorphous carbon films deposited in layer structure", APPLIED PHYSICS LETTERS, AIP, AMERICAN INSTITUTE OF PHYSICS, MELVILLE, NY, US, vol. 73, no. 2, 13 July 1998 (1998-07-13), pages 184-186, XP012021375, ISSN: 0003-6951, DOI: 10.1063/1.121749
- VOEVODIN A A ET AL: "Design of a Ti/TiC/DLC functionally gradient coating based on studies of structural transitions in Ti-C thin films", THIN SOLID FILMS, ELSEVIER-SEQUOIA S.A. LAUSANNE, CH, vol. 298, no. 1-2, 20 April 1997 (1997-04-20), pages 107-115, XP004125924, ISSN: 0040-6090, DOI: 10.1016/S0040-6090(96)09145-6

## Description

The present invention relates to the material preparation field, particularly to a device for preparation of nano-multilayer film and the preparation method thereof.

Currently, the vacuum magnetron sputtering technology has been extensively applied in various fields. One of the applications is coating the surface of a substrate, e.g. the surfaces of such substrates as medical surgical equipment, human implantation medical materials and engineering tools, with the diamond-like carbon (DLC) by making use of the vacuum magnetron sputtering technology, to significantly increase hardness and wear resistance of the substrate.

However, the diamond-like carbon, having a bigger internal stress, has a binding force not high with metal or alloy materials, and is prone to rupture or peeling under the action of higher load or load impact; especially for the human implantation product, the peeling of diamond-like carbon may produce debris, which will aggravate wear of the implantation product and reduce the service life of the product.

In order to resolve the above problems, the present research provides a nano-multilayer film, which can increase the wear resistance of the substrate, increase the binding force with the substrate, and contribute to improvement of lubricity at the same time. For example, the presented carbon/titanium carbide nano-multilayer film is coated onto the surface of the metal articular head and acetabular cup of the human-implantation product, making the articular head and acetabular cup have excellent wear resistance and good biocompatibility, as well as good lubricity, significantly increasing the service life of the artificial joint in the human body.

However, there is a very high requirement for the power technology when processing the nano-multilayer film by making use of the current magnetron sputtering coating device and, due to the limitation of the current industrial system, it is very difficult to achieve precise control on the industrialized scale and to realize industrialized production of the multilayer film.

An apparatus of the prior art for physical vapour deposition is disclosed in German patent publication DE 10 2008 062 332 A1. The apparatus is equipped with vaporisers of differing compositions, and has a segregated treatment chamber such that substrates are only subjected to deposition from one vaporiser at a time.

A multilayer amorphous carbon film, and a method of achieving stress relaxation in thick amorphous carbon films is disclosed in Appl. Phys. Lett. 73, 184 (1998), NPL number XP012021375. Amorphous carbon films are deposited by sputtering with alternating positive and negative bias voltages to obtain layers alternating between being rich in *sp*² sites and *sp*³ sites.

An alternative multilayer amorphous carbon film is disclosed in United States patent publication US 2007/0111003 A1. The multilayer film comprises first an adhesive layer on a substrate. Above the adhesive layer are multiple layers of doped diamond-like carbon, above which is a layer of undoped diamond-like carbon. The amount of dopant decreases from the lower to the top layer.

A further multilayer film is disclosed in Chinese patent publication CN 102247620 A. This film includes alternating layers of carbon, and a mixture of carbon and titanium carbide. The film is applied by a process of magnetron sputtering using two graphite targets and two titanium targets arranged around a rotating gantry.

A film with a gradually changing composition is disclosed in Thin Solid Films vol. 298, nr. 1-2, pp. 107-115 (1998), NPL number XP004125924. An initial layer of *α*-Ti is applied to a substrate. Above this, a plurality of layers of titanium carbide in the form TiₓC_{y} are applied, with x, the titanium content, decreasing from 0.90 to 0.10 and y, the carbon content, increasing from 0.10 to 0.90. Finally a layer of diamond-like carbon is applied. The resulting film resists brittle failure tests.

A purpose of the present invention is to at least improve upon the proposals set forth in the prior art in terms of the device for preparation of nano-multilayer film, and the preparation method thereof.

Thus according to an aspect of the present invention, according to claim 1, there is provided a magnetron sputtering coating device comprising a deposition chamber (100), a rotating stand (102) on a base of the deposition chamber and having a central axis, a first rotation system for driving the rotating stand to rotate around its central axis, a work steady (140) on the rotating stand, a sputtering cathode including two first sputtering cathodes (120a, 120b) located on and which equally divide a circumference concentric with the central axis of the rotating stand, and one second sputtering cathode (130) located on said circumference and which equally divides the arc between the two first sputtering cathodes, wherein the two first sputtering cathodes are the same chemical element, which is a different to the second sputtering cathode.

The magnetron sputtering coating device is characterized in that it further comprises a baffle (110) fixedly arranged on the rotating stand through its central axis to equally divide the rotating stand into two separate areas (102-1, 102-2), the baffle having two ends which extend beyond both ends of the sputtering cathode in the direction of the central axis of the rotating stand.

Optionally, the two first sputtering cathodes are graphite targets, and the second sputtering cathode is a titanium or tantalum target.

Optionally, the baffle has a width greater than the diameter of the rotating stand.

Optionally, the baffle is 2-10 cm away from the circumference where the sputtering cathode is located.

Optionally, the sputtering cathode is rectangular.

Optionally, the baffle is made of titanium, aluminium, stainless steel or the combination thereof.

Optionally, it further comprises a second rotation system for driving the work steady to rotate around a central axis of the work steady.

Optionally, the work steady is arranged on the rotating stand through a support rod, the same of which being provided with multiple work steadies at intervals.

Optionally, the sputtering cathode is arranged on an inner wall of the deposition chamber.

Optionally, the sputtering cathode further includes another second sputtering cathode located on said circumference opposite to the other second sputtering cathode, such that the four sputtering cathodes equally divide said circumference.

In another aspect of the present invention, there is provided a method of applying a nano-multilayer film to a substrate using the above magnetron sputtering coating device, comprising a first sputtering stage, a second sputtering stage and a third sputtering stage. The method is characterized in that:
(i) in the first sputtering stage:
   the working current of two first sputtering cathodes (120a, 120b) is set to zero,
   the working current of the second sputtering cathode (130) is set to a first predetermined current value and is set to constant voltage mode,
   the working bias voltage is kept at first predetermined bias voltage, and then
   sputtering of a first predetermined duration is performed, and then
   all sputtering cathodes are set to constant voltage mode,
   the working bias voltage is kept at a second predetermined bias voltage,
   the working current of the two first sputtering cathodes, starting from an initial working current 11, is increased by ΔI1 at intervals of a first time interval T1, until reaching a second predetermined current value,
   the working current of the second sputtering cathode, starting from the first predetermined current value, is decreased by ΔI2 at intervals of a second time interval T2, until reaching a third predetermined current value;
(ii) in the second sputtering stage:
   all sputtering cathodes are set to constant voltage mode,
   the working bias voltage is kept at the second predetermined bias voltage,
   the working current of the second sputtering cathode is set to and kept at a fourth predetermined current value,
   the working current of the two first sputtering cathodes is kept at the second predetermined current value or is set to and kept at a fifth predetermined current value, and then
   sputtering of a second predetermined duration is performed;
(iii) in the third sputtering stage:
   the working bias voltage is kept at the second predetermined bias voltage,
   the working current and voltage of the second sputtering cathode are set to zero,
   the two first sputtering cathodes are kept at the working current of the second sputtering stage or are set to and kept at a sixth predetermined current value, and are kept in constant voltage mode, and then
   sputtering of a third predetermined duration is performed; and
(iv) the rotating stand is rotated uniformly throughout the three sputtering stages.

Optionally, the first predetermined current value is 3.0-7.0 A, voltage of the constant voltage mode is 800 V. the first predetermined bias voltage is 100-200 V, the first predetermined duration is 5-15 min; the initial working current I1 is 0.5-2.0 A, the first time interval T1 is 5-15 min, ΔI1 is 0.5-1.5 A, the second predetermined current value is 4.0-7.0 A, the second time interval T2 is 5-15 min, ΔI2 is 0.5-1.5 A, the third predetermined current value is 0-3.0 A, the second predetermined bias voltage is 60-130 V.

Optionally, the fourth predetermined current value is 0.5-2.0 A, and the second predetermined duration is 120-400 min, the fifth predetermined current value is 4.0-7.0 A.

Optionally, the sixth predetermined current value is 4.0-7.0 A, and the third predetermined duration is 15-20 min.

Optionally, a step of cleaning the sputtering cathode is further included before the first sputtering stage.

Compared with the prior art, the magnetron sputtering coating device of the present invention equally divides the rotating stand into two mutually independent areas by arranging a baffle on the rotating stand. Because both ends of the baffle are beyond both ends of the sputtering cathode in the direction perpendicular to the rotating stand, the product to be coated at different areas, adapted to the position set for the sputtering cathode and at a certain position in the rotation of the rotating stand, only accepts coating from the first sputtering cathode and the second sputtering cathode, respectively, or only from the first sputtering cathode. Thus the nano-multilayer film is prepared by the rotation of the rotating stand. The device is simple in structure and process control, and realizes preparation of the nano-multilayer film, suitable for industrialization.

By making use of the magnetron sputtering coating device and method of the present invention, through controlling the working current and voltage at different sputtering stage, the nano-multilayer film is formed, including the transition layer, the composite layer of alternately laminated diamond-like carbon and quasi-graphite layers, and the diamond-like carbon top-layer film, having better binding force, lubricity and wear resistance.

The above and/or additional aspects and advantages of the present invention will become obvious and comprehensible from the following description of the examples with reference to the drawings.
Fig. 1 is a stereoscopic structural schematic view of the magnetron sputtering coating device according to the example of the present invention.
Fig. 2 is a top schematic view of the magnetron sputtering coating device as shown in Fig. 1.
Fig. 3 is a top schematic view of a magnetron sputtering coating device not forming part of the present invention.
Fig. 4 is a sectional structural schematic view of the nano-multilayer film in combination with the metal material according to the example of the present invention.

For better understanding the technical solution and beneficial effects of the present invention, the examples of the present invention will be described in detail below. The examples described below with reference to the drawings are exemplary only for explanation rather than restriction of the present invention.

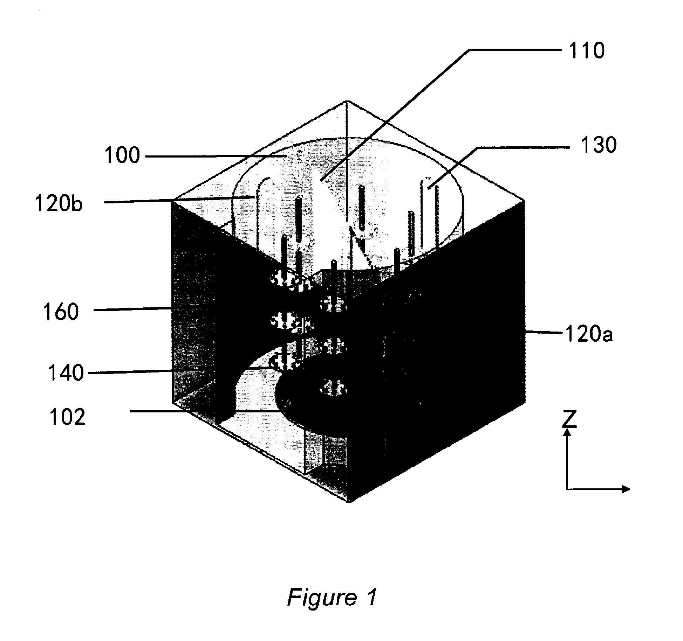

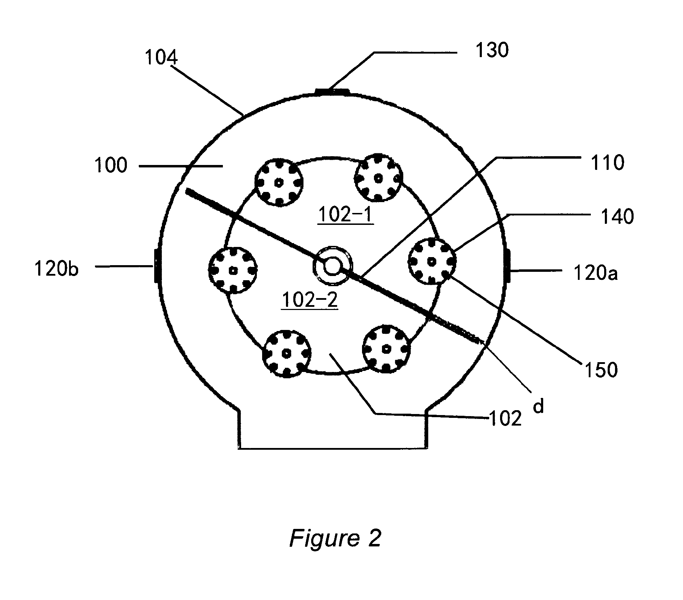

As shown in Fig. 1 and Fig. 2, Fig. 1 and Fig. 2 is a stereoscopic and top structural schematic view of the magnetron sputtering coating device according to the example of the present invention. The magnetron sputtering coating device in the example includes a deposition chamber 100, a rotating stand 102, a sputtering cathode, and a work steady 140, as well as a first rotation system (not shown in the drawing) for driving the rotating stand 102 to rotate around a central axis of the rotating stand; the device certainly further includes other necessary parts, such as a heating device, a temperature control system, a cooling water circulation system, and a power supply system electrically connected with the sputtering cathode (not shown in the drawing).

In the example, the sputtering cathode includes two first sputtering cathodes 120a and 120b and one second sputtering cathode 130, which are arranged on the circumference 104 concentric with the rotating stand 102; the circumference where the sputtering cathode is located can either be an actual part such as the inner wall of the deposition chamber 100, or be a virtual circumference such as any position between the rotating stand and the deposition chamber, where the two first sputtering cathodes 120a and 120b are arranged relatively parallel to each other and equally divide the circumference 104, while the second sputtering cathode 130 equally divides the arc between the two first sputtering cathodes 120a and 120b; that is, the arc between the two first sputtering cathodes 120a and 120b is substantially of 180°, while the arc between the second sputtering cathode 130 and the first sputtering cathodes 120a and 120b is substantially of 90°; the first sputtering cathodes 120a and 120b can be a sputtering cathode of a certain chemical element, while the second sputtering cathode can be a sputtering cathode of another chemical element. The sputtering cathode material can be selected according to the specific product needing to be coated; for example, in the example the first sputtering cathode is a graphite target while the second sputtering cathode a titanium target, and in other examples the first sputtering cathode can be a graphite target while the second sputtering cathode a tantalum target, etc.

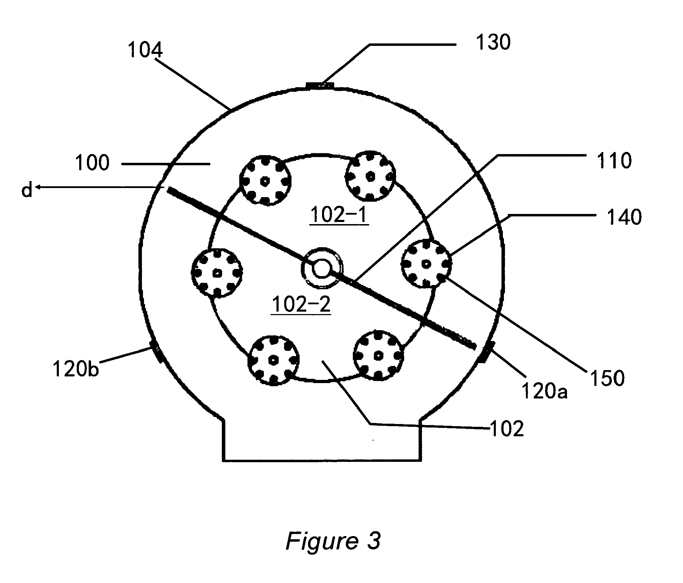

In other examples not forming part of the present invention, the two first sputtering cathodes and one second sputtering cathode can also be arranged on the circumference at intervals at other angles; for example, the arc (superior arc) between the two first sputtering cathodes 120a and 120b is of 180-240°, while the one second sputtering cathode equally divides the arc; therefore, the arc between the second sputtering cathode and the first sputtering cathode can be of 90-120°, as shown in Fig. 3, while the arc between the second sputtering cathode 130 and the first sputtering cathodes 120a and 120b is of 120°.

In the example of the present invention, the rotating stand 102 is a circular platform, on which is fixed a baffle 110; the baffle 110, being preferably a straight plate, can be made of titanium, aluminium, stainless steel and the like or the combination thereof; the baffle 110 goes through the diameter of the rotating stand and is arranged perpendicular to the rotating stand 102, dividing the rotating stand 102 into two mutually independent areas 102-1 and 102-2; both ends of the baffle 110, in the direction perpendicular to the rotating stand (the direction of the Z axis), are beyond both ends of the sputtering cathodes 120a, 120b and 130; therefore, the baffle blocks the other face of the sputtering cathode in a certain area, making the area only accept the coating of the sputtering cathode opposing the area; more preferably, for achieving a better blocking effect, the baffle has a width bigger than the diameter of the rotating stand, where the width refers to the length of the baffle going through the rotating stand in the diameter direction; more preferably, the distance d between the baffle and the circumference where the sputtering cathode is located is 2-10 cm.

The rotating stand 102 is provided with a first rotation system (not shown in the drawing) for driving the rotating stand to rotate around a central axis of the rotating stand; that is, the rotating stand and the baffle along with the work steady rotate around the central axis of the rotating stand. Under the condition of the above arrangement of the three sputtering cathodes, when the rotating stand rotates to any position, e.g. the position as shown in Fig. 2, with the blocking of the baffle 102, an area 102-1 of the rotating stand is opposed to the first sputtering cathode 120a (e.g. the graphite target) and the second sputtering cathode 130 (the titanium target), enabling the product (or the substrate) to be coated in the area 102-1 with a film of carbon:titanium carbide (a mixed layer of carbon with titanium carbide); while another area 102-2 of the rotating stand is opposed to the first sputtering cathode 120b (the graphite target), thus making the product in the area 102-2 coated with the carbon film; with rotation of the rotating stand, the product in different areas will be coated alternately with the carbon:titanium carbide film and the carbon film, thus realizing the coating of the product with the nano-multilayer film. The thickness of a single layer can be controlled by adjustment of the rotational speed of the rotating stand. The device is simple in structure and process control, and realizes preparation of the nano-multilayer film, suitable for industrialization.

In other examples not forming part of the present invention, the baffle 110 can also be arranged perpendicularly at other positions of the rotating stand, or be a bent plate or any other baffle that divides the rotating stand into two mutually independent areas.

In the example of the present invention, the device further has a second rotation system for driving the work steady to rotate around the central axis of the work steady; that is, the work steady can rotate on its axis. Multiple work steadies 140 are arranged on the rotating stand 102 through the support rod 160, and the same support rod 160 can be provided at intervals with multiple work steadies 140 to improve the processing efficiency; the work steady 140 is used to receive the substrate (or product) 150 to be processed, which can be uniformly arranged on the circumference of the work steady 140. With the rotation of the work steady on its axis, the coating on each of the products to be coated on the work steady can have good uniformity.

What is described above is the magnetron sputtering coating device of the preferred example of the present invention; to be different from the example, in another preferred example, there are four sputtering cathodes (not shown in the drawing) arranged around the rotating stand; that is, the sputtering cathode includes two first sputtering cathodes and two second sputtering cathodes, the two first sputtering cathodes arranged opposite to each other, the two second sputtering cathodes arranged opposite to each other, the four sputtering cathodes equally dividing the circumference. However, during the preparation of the multilayer film, one of the second sputtering cathodes does not work; that is, the corresponding parameters such as the target current and voltage are set for the two first sputtering cathodes and one of the second sputtering cathodes instead of for the other second sputtering cathode, which is not used for sputtering and in an idle state. In the example, although four sputtering cathodes are arranged, one of them is not used for sputtering.

In the present invention, the substrate to be coated can either be human implantation equipment such as bone articular head or acetabular cup, or be other substrates such as engineering items, the substrate being made of metal or alloy materials or other materials.

What is described above is a detailed description of the magnetron sputtering coating device of the present invention. During the preparation, as required specifically, each processing parameter is set for the preparation of the nano-multilayer film. For this, the present invention provides a method of coating the substrate by making use of any of the above magnetron sputtering coating devices, the method comprising three sputtering stages.

In a first sputtering stage, the working current of two first sputtering cathodes is set to zero. A second sputtering cathode is set to have a working current of first predetermined current value and to be at constant voltage mode, the working bias voltage is kept at first predetermined bias voltage and then the sputtering of a first predetermined duration is performed. Then, all sputtering cathodes are at constant voltage mode, the working bias voltage is kept at a second predetermined bias voltage. working current of the two first sputtering cathodes, starting from an initial working current I1, is increased by ΔI1 at intervals of a first time interval T1, until reaching a second predetermined current value; the working current of the one second sputtering cathode, starting from the first predetermined current value, is decreased by ΔI2 at intervals of a second time interval T2, until reaching a third predetermined current value.

In a second sputtering stage, all sputtering cathodes are at constant voltage mode, the working bias voltage is kept at the second predetermined bias voltage. the working current of the second sputtering cathode is set to and kept at a fourth predetermined current value, the working current of the first sputtering cathode is kept at the second predetermined current value or is set to and kept at a fifth predetermined current value, and then the sputtering of a second predetermined duration is performed.

In a third sputtering stage, working bias voltage is kept at the second predetermined bias voltage, the working current and voltage of the second sputtering cathode are set to zero, the first sputtering cathode is kept at the working current of the second sputtering stage or is set to and kept at a sixth predetermined current value, and is kept at the constant voltage mode, and then the sputtering of a third predetermined duration is performed.

In the examples of the preparation method of the present invention, three sputtering cathodes, i.e. two first sputtering cathodes and one second sputtering cathode, are used for the preparation; in the example further including the other second sputtering cathode, the second sputtering cathode is in an idle state and kept from being sputtered in the preparation of the nano-multilayer film.

In some examples, the first sputtering cathode of the magnetron sputtering coating device is the graphite target, while the second sputtering cathode is a titanium target. First, the substrate to be coated (or the product to be coated) is put on the work steady in the deposition chamber, and the deposition chamber is vacuumed to 2×10⁻⁴ Pa, with the high pure argon introduced at a flow of 25 sccm.

Then, the sputtering cathode can be cleaned, and all the sputtering cathodes can be applied with a current of 0.4 A and a voltage of 500 V for 20 minutes of sputter cleaning.

Then, the sputtering is performed. During the whole sputtering process, with the rotating stand rotated uniformly, the substrate to be coated can also be rotated on its axis.

More specifically, in the first sputtering stage, the first predetermined current value of the second sputtering cathode can be set at 3.0-7.0 A, voltage of the second sputtering cathode can be set at 800 V. The first predetermined bias voltage is 100-200 V, the first predetermined duration is 5-15 min.

Then, the working bias voltage can be set at the second predetermined of 60-130 V, wherein, the two first sputtering cathodes can be set at the initial working current I1 of 0.5-2.0 A. For the two first sputtering cathodes, at a time interval of such as 5-15 min, their working current is increased by a certain value such as 0.5-1.5 A, until their working current is increased to a predetermined value such as 4.0-7.0 A. On the contrary, for the one second sputtering cathode, at a time interval of 5-15 min, its working current is decreased by a certain value such as 0.5-1.5 A, until its working current is decreased to a predetermined value such as 0-3.0 A.

That is, during the sputtering process of the first stage, the current of the first sputtering cathode is increased stepwise, and the current of the second sputtering cathode is decreased stepwise, their increased and decreased amounts being possible to be identical or different, their time intervals being possible to be identical or different, their voltage being kept constant in the whole process;

Thus, after a period of sputtering such as for 50-100 min, the transition layer of 300-500 nm in thickness composed of titanium film and a mixed layer of TiC and quasi-graphite can be deposited onto the product of the metal material. Because the current gradients of different targets are controlled in the process, the mass percentage of Ti in the transition layer, in proximity to the metal material layer upwards, is decreased gradually from a higher content, while the mass percentage of C is increased gradually from a lower content, thus the binding force with the substrate, especially the substrates of metal or alloy materials, being increased, also its lubricity being increased gradually.

It is necessary to explain that, in the present invention the mixed layer of titanium carbide and quasi-graphite refers to a layer of titanium carbide mixed with the quasi-graphite; that is, the mixed layer contains quasi-graphite and titanium carbide at the same time.

Then, in the second sputtering stage, the working current of the first sputtering cathode can be kept at 4.0-7.0 A, which is the last predetermined value of the first sputtering stage. Certainly, the working current of the first sputtering cathode can also be set and kept to be reset and kept at a desired predetermined value such as 4.0-7.0 A. Meanwhile, the working current of the second sputtering cathode is set and kept at a desired predetermined value such as 0.5-2.0 A, voltage of all sputtering cathodes is kept at constant voltage of 800 V. Sputtering is performed for 120-400 min, during which the working bias voltage is kept constant of the second predetermined bias voltage. Under these processing conditions, a composite layer of 1600-3300 nm is formed, which is a multilayer structure with the quasi-graphite layer (the content of sp2 is about 60 %) and the diamond-like carbon layer (the content of sp3 is about 70 %) alternately laminated.

Then, in the third sputtering stage, the working bias voltage is kept at constant of the second predetermined bias voltage. The voltage and current of the second sputtering cathode are decreased to zero, making it idle, and the current of the two first sputtering cathodes is reset to a desired predetermined value such as 4.0-7.0 A, and can certainly also be kept at the current of the second sputtering stage. Voltage is kept at constant voltage of 800 V, and sputtering is performed for 15-20 min. Under these processing conditions, a diamond-like carbon top-layer film of about 100 nm is separately formed on the composite layer, the quasi-diamond of high hardness making the nano-multilayer film have better hardness and wear resistance. So far a nano-multilayer film in combination with the substrate is formed; in the example, the mass percentage of C in the formed nano-multilayer film is 70-97.6 %, and the mass percentage of Ti is 2.4-30 %.

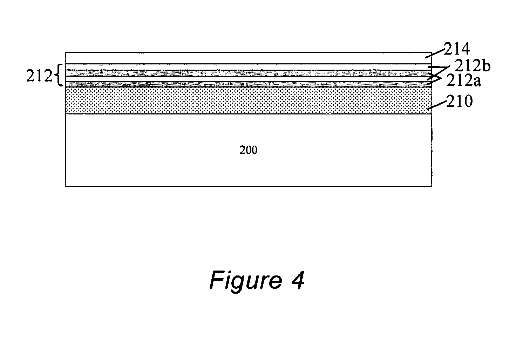

The nano-multilayer film formed by the above method with the above device that is in combination with the substrate, is shown in Fig. 4, which includes the transition layer 210 composed of Ti film and a mixed layer of TiC and quasi-graphite on the substrate 200, the composite layer 212 of alternately laminated diamond-like carbon and quasi-graphite on the transition layer 210, and the diamond-like carbon top-layer film 214 on the composite layer 212. The content of sp2 in the quasi-graphite layer is 60 % and the content of sp3 in the diamond-like carbon layer is 70 %. In the direction from the substrate 200 to the diamond-like carbon top-layer film 214, the mass percentage of Ti in the mixed layer is gradually decreased, while the mass percentage of C is gradually increased. The composite layer 212 is a multilayer structure with the quasi-graphite layer 212a and the diamond-like carbon layer 212b alternately laminated. There is no limit to the number of the quasi-graphite layer and the diamond-like carbon layer in the composite layer 212. The interface of the nano-multilayer film where the transition layer is in combination with the substrate contains higher content of titanium and lower content of carbon, having a higher binding force with the substrate, also having low internal stress and good lubricity, thus the hardness and wear resistance being improved through the diamond-like carbon top-layer.

### Example 1

In a specific example 1, the two first sputtering cathodes are graphite targets, while the one second sputtering cathode is a titanium target. The arc between the second sputtering cathode and the first sputtering cathode in the sputtering device is substantially of 90°, the rotating stand has a rotational speed of 1.5 rpm, and the substrate to be coated is rotated on its axis. Before coating, the substrate to be coated is put onto the work steady of the deposition chamber, and the deposition chamber is vacuumed to 2×10⁻⁴ Pa, with the high pure argon introduced at a flow of 25 sccm.

Then, all the sputtering cathodes are applied with a current of 0.4 A and a voltage of 500 V for 20 minutes of sputter cleaning.

Then the sputtering is performed, graphite targets do not work first. Working bias voltage is set to 150 V, one titanium target being set to have a constant voltage of 800 V and a current of 7.0 A, and sputtering of 10 min is performed.

Then, the working bias voltage is decreased to 100 V. The titanium target is kept at constant voltage of 800 V and having a current decreased stepwise by 1.0 A at intervals of 10 min until down to zero. The two graphite targets are set to have constant voltage of 800 V and a working current started from 1.5 A and increased stepwise by 1.0 A at intervals of 10 min until up to 7.0 A. After sputtering of 60 min, a transition layer composed of titanium film and a mixed layer of TiC and quasi-graphite (the ratio of C of the mixed layer is increased gradually, while the ratio of Ti is decreased gradually) of 300 nm is deposited onto the substrate;.

Then, with the current and voltage of the two graphite targets kept constant, the current of the titanium target is increased to 1.0 A, with the voltage kept at constant voltage. The working bias voltage is still 100 V. After 120 min, a composite layer of 1600 nm is formed on the transition layer, comprising the quasi-graphite layer (the content of sp2 is about 60 %) and the diamond-like carbon layer (the content of sp3 is about 70%) alternately laminated.

Then the current and voltage of the titanium target are decreased to zero, that is to say does not work, and the current of the graphite target is increased to 7.0 A, with the voltage kept at 800 V. The working bias is kept at 100 V. fter 15 min, a diamond-like carbon film of 100 nm is formed on the composite layer. In the example, the mass percentage of C in the formed nano-multilayer film is 97 %, and the mass percentage of Ti is 3 %.

### Example 2

In another specific example 2, the two first sputtering cathodes in the sputtering device are graphite targets, while the one second sputtering cathode is a titanium target. The arc between the second sputtering cathode and the first sputtering cathode is substantially of 90°, the rotating stand has a rotational speed of 2.0 rpm, and the substrate to be coated is rotated on its axis. Before coating, the substrate to be coated is put onto the work steady of the deposition chamber, and the deposition chamber is vacuumed to 2×10⁻⁴ Pa, with the high pure argon introduced at a flow of 25 sccm.

Then, all the sputtering cathodes are applied with a current of 0.4 A and a voltage of 500 V for 20 minutes of sputter cleaning.

Then the sputtering is performed, graphite targets do not work first. Working bias voltage is set to 120 V. One titanium target is set to have a constant voltage of 800 V and a current of 6.5 A, and sputtering of 5 min is performed.

Then, working bias voltage is decreased to 110 V, and the titanium target is kept at constant voltage of 800 V and having a current decreased stepwise by 1.0 A at intervals of 5 min until down to zero. The two graphite targets are set to have constant voltage of 800 V and a working current started from 0.8 A and increased stepwise by 1.0 A at intervals of 5 min until up to 6.5 A. After 80 min, a transition layer composed of titanium film and a mixed layer of TiC and quasi-graphite (the ratio of C of the mixed layer is increased gradually, while the ratio of Ti is decreased gradually) of 400 nm is deposited onto the substrate.

Then, with the current and voltage of the two graphite targets kept constant, the current of the titanium target is increased to 1.5 A, with the voltage kept at constant voltage. The working bias voltage is still 110 V. After 180 min, a composite layer of 1800 nm is formed on the transition layer, comprising the quasi-graphite layer (the content of sp2 is about 60 %) and the diamond-like carbon layer (the content of sp3 is about 70 %) alternately laminated.

Then the current and voltage of the titanium target are decreased to zero. The current of the graphite target is increased to 6.5 A, with the voltage kept at 800 V. The working bias is kept at 110 V. After 15 min, a diamond-like carbon film of 100 nm is formed on the composite layer. In the example, the mass percentage of C in the formed nano-multilayer film is 92 %, and the mass percentage of Ti is 8 %.

### Example 3

In still another specific example 3, the two first sputtering cathodes in the sputtering device are the graphite targets, while the one second sputtering cathode is a titanium target. The arc between the second sputtering cathode and the first sputtering cathode is substantially of 90°, the rotating stand has a rotational speed of 2.5 rpm, and the substrate to be coated is rotated on its axis. Before coating, the substrate to be coated is put onto the work steady of the deposition chamber, and the deposition chamber is vacuumed to 2×10⁴ Pa, with the high pure argon introduced at a flow of 25 sccm.

Then, all the sputtering cathodes are applied with a current of 0.4 A and a voltage of 500 V for 20 minutes of sputter cleaning.

Then the sputtering is performed, graphite targets do not work first. Working bias voltage is set to 150 V. One titanium target is set to have a constant voltage of 800 V and a current of 6.0 A, and sputtering of 12 min is performed.

Then, a working bias voltage is decreased to 120 V, and the titanium target is kept at constant voltage of 800 V and having a current decreased stepwise by 1.0 A at intervals of 12 min until down to zero. The two graphite targets are set to have constant voltage of 800 V and a working current started from 1.8 A and increased stepwise by 1.0 A at intervals of 12 min until up to 6.0 A. After 100 min, a transition layer composed of titanium film and a mixed layer of TiC and quasi-graphite (the ratio of C of the mixed layer is increased gradually, while the ratio of Ti is decreased gradually) of 500 nm is deposited onto the substrate.

Then, with the current and voltage of the two graphite targets kept constant, the current of the titanium target is increased to 1.8 A, with the voltage kept at constant voltage. The working bias voltage is still 120 V. After 260 min, a composite layer of 1900 nm is formed on the transition layer, comprising the quasi-graphite layer (the content of sp2 is about 60 %) and the diamond-like carbon layer (the content of sp3 is about 70 %) alternately laminated.

Then the current and voltage of the titanium target are decreased to zero. The current of the graphite target is increased to 6.0 A, with the voltage kept at 800 V. Working bias is kept at 120 V. After 15 min, a diamond-like carbon film of 100 nm is formed on the composite layer. In the example, the mass percentage of C in the formed nano-multilayer film is 86 %, and the mass percentage of Ti is 14 %.

An observation is made to the surface of the nano-multilayer film of the above specific examples 1-3 by the scanning electron microscope, finding that the surface of the nano-multilayer film has small surface roughness. The hardness of the nano-multilayer film is measured by a nanoindentation instrument, with a load of 10 mN, the pressed depth being greater than 10 times of the surface roughness and smaller than 1/10 of the thickness of the nano-multilayer film, so as to guarantee the authenticity and validity of the measured hardness. The longitudinal binding force and the lateral binding force of the nano-multilayer film are evaluated through the standard indentation instrument and the automatic scratch test. With the load of the indentation test being 150 N and the radius of the indenter being 0.2 mm, no crack and delamination is found around the indentation, indicating that the nano-multilayer film has a high longitudinal binding force. The load is increased gradually from 10 N to 85 N, with a sliding velocity of 10 mm/min. There is no crack and peeling around and at the edge of the scratch, indicating that the nano-multilayer film has a high lateral binding force.

The ball-disc friction and wear machine is used for determination of the friction and wear properties of the nano-multilayer film. The grinding ball is a Si3N4 ceramic ball having a hardness of 1500 HV and a diameter of 3 mm. The load is 10 N, the sliding velocity is 0.10 m/s, and the friction duration is 30 min. The experiments are made under the conditions of room temperature, no lubrication, and humid air (50 % relative humidity). In the testing process, the change of the friction coefficient is automatically recorded. After the test, the wear volume is measured with a step meter, with the wear rate calculated. Table 1 shows the hardness, average friction coefficient and wear rate of the nano-multilayer film of Examples 1, 2 and 3.

**Table 1**

| Nano-multilayer film | Content of Ti (at. %) | Sectional binding force of nano-multilayer film with substrate (N) | | Average friction coefficient (*µ*) | Wear rate (m³/Nm) |
|---|---|---|---|---|---|
| | | Longitudinal | Lateral | | |
| Example 1 | 3 | >150 | ≧5 | 0.048 | 2.8×10⁻¹⁷ |
| Example 2 | 8 | >150 | ≧5 | 0.052 | 2.3×10⁻¹⁷ |
| Example 3 | 14 | >150 | ≧5 | 0.046 | 3.1×10⁻¹⁷ |

The nano-multilayer film of the present invention has a low friction coefficient, super wear resistance and a high bonding force with the substrate of the product. In the nano-multilayer film of the specific examples 1, 2 and 3, the average friction coefficients of their films are 0.048, 0.052 and 0.046, respectively, without obvious change of the friction coefficient during the test process, indicating that the nano-multilayer film has good friction stability, the surface wear trace of the nano-multilayer film being very shallow after the wear; the wear rates of the nano-multilayer films of the specific examples 1, 2 and 3 are 2.8×10⁻¹⁷, 2.3×10⁻¹⁷ and 3.1×10⁻¹⁷ m³/Nm, respectively, indicating that the nano-multilayer film has very good wear resistance. Moreover, the pits of the nano-multilayer films of the specific examples 1, 2 and 3 formed under the load of 150 N have no crack, delamination or peeling trace at the edge thereof; under the load of 85 N, there is no crack or peeling of the nano-multilayer film observed in the area and at the edge of the scratch formed on the surface of the nano-multilayer film. The pit and scratch tests show that the nano-multilayer film has excellent bonding strength with the metal substrate.

What is described above is only the preferred embodiments of the present invention. It should be pointed out that, for those of ordinary skill in the art, some improvement and amendment can further be made under the premise of not departing from the principles of the present invention, and should also be regarded as being within the scope of protection of the present invention.

## Claims

1. A magnetron sputtering coating device comprising:
a deposition chamber (100);
a rotating stand (102) on a base of the deposition chamber and having a central axis;
a first rotation system for driving the rotating stand to rotate around its central axis;
a work steady (140) on the rotating stand;
a sputtering cathode including two first sputtering cathodes (120a, 120b) located on and which equally divide a circumference (104) concentric with the central axis of the rotating stand, and one second sputtering cathode (130) located on said circumference and which equally divides the arc between the two first sputtering cathodes;
wherein the two first sputtering cathodes are the same chemical element, which is a different element to the second sputtering cathode;
**characterized in that** the device further comprises a baffle (110) fixedly arranged on the rotating stand through its central axis to equally divide the rotating stand into two separate areas (102-1, 102-2), the baffle having two ends which extend beyond both ends of the sputtering cathode in the direction of the central axis of the rotating stand.

2. The magnetron sputtering coating device according to claim 1, **characterized in that** the two first sputtering cathodes (120a, 120b) are graphite targets, and the second sputtering cathode (130) is a titanium or tantalum target.

3. The magnetron sputtering coating device according to claim 1, **characterized in that** the baffle (110) has a width greater than the diameter of the rotating stand.

4. The magnetron sputtering coating device according to claim 3, **characterized in that** the baffle (110) is 2-10 cm away from the circumference (104) where the sputtering cathode (120a, 120b, 130) is located.

5. The magnetron sputtering coating device according to claim 1, **characterized in that** the sputtering cathode is rectangular.

6. The magnetron sputtering coating device according to claim 1, **characterized in that** the baffle (110) is made of titanium, aluminium, stainless steel or a combination thereof.

7. The magnetron sputtering coating device according to claim 1, **characterized in that** it further includes a second rotation system for driving the work steady (140) to rotate around a central axis of the work steady.

8. The magnetron sputtering coating device according to claim 7, **characterized in that** the work steady (140) is arranged on the rotating stand (102) through a support rod (160), the same of which being provided with multiple work steadies at intervals.

9. The magnetron sputtering coating device according to claim 1, **characterized in that** the sputtering cathode (120a, 120b, 130) is arranged on an inner wall of the deposition chamber.

10. The magnetron sputtering coating device according to claim 1, **characterized in that** the sputtering cathode further includes another second sputtering cathode located on said circumference (104) opposite to the other second sputtering cathode (130), such that the four sputtering cathodes equally divide said circumference.

11. A method of coating a substrate (150) with a nano-multilayer film, comprising a first sputtering stage, a second sputtering stage and a third sputtering stage, **characterized by** using the magnetron sputtering coating device of claim 1 to coat the substrate, wherein:
(i) in the first sputtering stage:
the working current of the two first sputtering cathodes is set to zero,
the working current of the second sputtering cathode is set to a first predetermined current value and is set to constant voltage mode,
the working bias voltage is kept at first predetermined bias voltage, and then
sputtering of a first predetermined duration is performed, and then
all sputtering cathodes are set to constant voltage mode,
the working bias voltage is kept at a second predetermined bias voltage,
the working current of the two first sputtering cathodes, starting from an initial working current I1, is increased by ΔI1 at intervals of a first time interval T1, until reaching a second predetermined current value;
the working current of the second sputtering cathode, starting from the first predetermined current value, is decreased by ΔI2 at intervals of a second time interval T2, until reaching a third predetermined current value;
(ii) in the second sputtering stage:
all sputtering cathodes are set to constant voltage mode,
the working bias voltage is kept at the second predetermined bias voltage,
the working current of the second sputtering cathode is set to and kept at a fourth predetermined current value,
the working current of the two first sputtering cathodes is kept at the second predetermined current value or is set to and kept at a fifth predetermined current value, and then
sputtering of a second predetermined duration is performed;
(iii) in the third sputtering stage:
the working bias voltage is kept at the second predetermined bias voltage,
the working current and voltage of the second sputtering cathode are set to zero,
the two first sputtering cathodes are kept at the working current of the second sputtering stage or are set to and kept at a sixth predetermined current value, and are kept in constant voltage mode, and then
sputtering of a third predetermined duration is performed: and
(iv) the rotating stand is rotated uniformly throughout said three sputtering stages.

12. The method according to claim 11, **characterized in that**:
the first predetermined current value is 3.0-7.0 A;
the voltage of the constant voltage mode is 800 V;
the first predetermined bias voltage is 100-200 V;
the first predetermined duration is 5-15 min;
the initial working current I1 is 0.5-2.0 A;
the first time interval T1 is 5-15 min;
ΔI1 is 0.5-1.5 A;
the second predetermined current value is 4.0-7.0 A;
the second time interval T2 is 5-15 min;
ΔI2 is 0.5-1.5 A;
the third predetermined current value is 0-3.0 A; and
the second predetermined bias voltage is 60-130 V.

13. The method according to claim 11, **characterized in that:**
the fourth predetermined current value is 0.5-2.0 A;
the second predetermined duration is 120-400 min; and
the fifth predetermined current value is 4.0-7.0 A.

14. The method according to claim 11, **characterized in that:**
the sixth predetermined current value is 4.0-7.0 A; and
the third predetermined duration is 15-20 min.

15. The method according to claim 11, **characterized in that** a step of cleaning the sputtering cathode is further included before the first sputtering stage.

## Patentansprüche

1. Eine Magnetron-Sputter-Beschichtungsvorrichtung, bestehend aus:
einer Abscheidekammer (100);
einem auf dem Boden der Abscheidekammer befindlichen und mit einer Mittelachse versehenen drehbaren Stand (102);
einem ersten Drehsystem für den Antrieb des drehbaren Standes zur Drehung um seine Mittelachse;
einer Lünette (140) auf dem drehbaren Stand;
einer Zerstäubungskathode einschließlich zweier erster Zerstäubungskathoden (120a, 120b), die sich auf einem mit der Mittelachse des drehbaren Standes konzentrischen Kreisumfang (104) befinden und diesen zu gleichen Teilen aufteilen, und einer zweiten Zerstäubungskathode (130), die sich auf besagtem Kreisumfang befindet und den Bogen zwischen den ersten beiden Zerstäubungskathoden zu gleichen Teilen aufteilt;
wobei die beiden ersten Zerstäubungskathoden aus demselben chemischen Element sind, welches ein anderes Element als das der zweiten Zerstäubungskathode ist;
**dadurch gekennzeichnet, dass** die Vorrichtung des Weiteren ein Prallblech (110) beinhaltet, das auf dem drehbaren Stand durch seine Mittelachse hindurch fest angebracht ist, so dass der drehbare Stand zu gleichen Teilen in zwei getrennte Bereiche (102-1, 102-2) unterteilt wird, wobei das Prallblech zwei Endseiten hat, die sich über beide Enden der Zerstäubungskathoden hinaus in Richtung der Mittelachse des drehbaren Standes erstrecken.

2. Die Magnetron-Sputter-Beschichtungsvorrichtung gemäß Patentanspruch 1, **dadurch gekennzeichnet, dass** die ersten beiden Zerstäubungskathoden (120a, 120b) Graphittargets sind und die zweite Zerstäubungskathode (130) ein Titan- oder Tantaltarget ist.

3. Die Magnetron-Sputter-Beschichtungsvorrichtung gemäß Patentanspruch 1, **dadurch gekennzeichnet, dass** das Prallblech (110) eine Breite aufweist, die über dem Durchmesser des drehbaren Standes liegt.

4. Die Magnetron-Sputter-Beschichtungsvorrichtung gemäß Patentanspruch 3, **dadurch gekennzeichnet, dass** das Prallblech (110) 2-10 cm vom Kreisumfang (104), an dem sich die Zerstäubungskathode (120a, 120b, 130) befindet, entfernt ist.

5. Die Magnetron-Sputter-Beschichtungsvorrichtung gemäß Patentanspruch 1, **dadurch gekennzeichnet, dass** die Zerstäubungskathode rechteckig ist.

6. Die Magnetron-Sputter-Beschichtungsvorrichtung gemäß Patentanspruch 1, **dadurch gekennzeichnet, dass** das Prallblech (110) aus Titan, Aluminium, Edelstahl oder einer Verbindung dieser besteht.

7. Die Magnetron-Sputter-Beschichtungsvorrichtung gemäß Patentanspruch 1, **dadurch gekennzeichnet, dass** sie des Weiteren ein zweites Drehsystem für den Antrieb der Lünette (140) zur Drehung um eine Mittelachse der Lünette beinhaltet.

8. Die Magnetron-Sputter-Beschichtungsvorrichtung gemäß Patentanspruch 7, **dadurch gekennzeichnet, dass** die Lünette (140) auf dem drehbaren Stand (102) durch eine Stativstange (160) angebracht ist, wobei selbige in Abständen mit mehreren Lünetten ausgestattet ist.

9. Die Magnetron-Sputter-Beschichtungsvorrichtung gemäß Patentanspruch 1, **dadurch gekennzeichnet, dass** die Zerstäubungskathode (120a, 120b, 130) auf einer Innenwand der Abscheidekammer angebracht ist.

10. Die Magnetron-Sputter-Beschichtungsvorrichtung gemäß Patentanspruch 1, **dadurch gekennzeichnet, dass** die Zerstäubungskathode des Weiteren eine andere zweite Zerstäubungskathode enthält, die sich auf besagtem Kreisumfang (104) gegenüber der anderen zweiten Zerstäubungskathode (130) befindet, so dass die vier Zerstäubungskathoden besagten Kreisumfang zu gleichen Teilen aufteilen.

11. Ein Verfahren für die Beschichtung eines Substrats (150) mit einer Nano-Mehrschichtfolie, bestehend aus einer ersten Zerstäubungsstufe, einer zweiten Zerstäubungsstufe und einer dritten Zerstäubungsstufe, **gekennzeichnet durch** die Verwendung der Magnetron-Sputter-Beschichtungsvorrichtung aus Patentanspruch 1 für die Beschichtung des Substrats, wobei:
(i) in der ersten Zerstäubungsstufe:
der Betriebsstrom der beiden ersten Zerstäubungskathoden auf null gesetzt wird,
der Betriebsstrom der zweiten Zerstäubungskathode auf einen ersten vorbestimmten Stromwert eingestellt wird und in Konstantspannungsbetrieb versetzt wird,
die Betriebsvorspannung auf der ersten vorbestimmten Vorspannung gehalten wird, und dann
Sputtern von einer ersten vorbestimmten Zeitdauer erfolgt, und dann
alle Zerstäubungskathoden in Konstantspannungsbetrieb versetzt werden,
die Betriebsvorspannung auf einer zweiten vorbestimmten Vorspannung gehalten wird,
der Betriebsstrom der beiden ersten Zerstäubungskathoden ausgehend von einem ursprünglichen Betriebsstrom 11 in Abständen von einem ersten Zeitabstand T1 um ΔI1 erhöht wird, bis er einen zweiten vorbestimmten Stromwert erreicht;
der Betriebsstrom der zweiten Zerstäubungskathode ausgehend vom ersten vorbestimmten Stromwert in Abständen von einem zweiten Zeitabstand T2 um ΔI2 gesenkt wird, bis er einen dritten vorbestimmten Stromwert erreicht;
(ii) in der zweiten Zerstäubungsstufe:
alle Zerstäubungskathoden in Konstantspannungsbetrieb versetzt werden,
die Betriebsvorspannung auf der zweiten vorbestimmten Vorspannung gehalten wird,
der Betriebsstrom der zweiten Zerstäubungskathode auf einen vierten vorbestimmten Stromwert eingestellt und darauf gehalten wird,
der Betriebsstrom der beiden ersten Zerstäubungskathoden auf dem zweiten vorbestimmten Stromwert gehalten wird oder auf einen fünften vorbestimmten Stromwert eingestellt und darauf gehalten wird, und dann
Sputtern von einer zweiten vorbestimmten Zeitdauer erfolgt;
(iii) in der dritten Zerstäubungsstufe:
die Betriebsvorspannung auf der zweiten vorbestimmten Vorspannung gehalten wird,
der Betriebsstrom und die Betriebsspannung der zweiten Zerstäubungskathode auf null gesetzt werden,
die beiden ersten Zerstäubungskathoden auf dem Betriebsstrom der zweiten Zerstäubungsstufe gehalten werden oder auf einen sechsten vorbestimmten Stromwert eingestellt und darauf gehalten werden und in Konstantspannungsbetrieb verbleiben, und dann
Sputtern von einer dritten vorbestimmten Zeitdauer erfolgt; und
(iv) der drehbare Stand während besagter drei Zerstäubungsstufen gleichmäßig gedreht wird.

12. Das Verfahren gemäß Patentanspruch 11, **dadurch gekennzeichnet, dass:**
der erste vorbestimmte Stromwert 3,0-7,0 A beträgt;
die Spannung im Konstantspannungsbetrieb 800 V beträgt;
die erste vorbestimmte Vorspannung 100-200 V beträgt;
die erste vorbestimmte Zeitdauer 5-15 min beträgt;
der ursprüngliche Betriebsstrom I1 0,5-2,0 A beträgt;
der erste Zeitabstand T1 5-15 min beträgt;
ΔI1 0,5-1,5 A beträgt;
der zweite vorbestimmte Stromwert 4,0-7,0 A beträgt;
der zweite Zeitabstand T2 5-15 min beträgt;
ΔI2 0,5-1,5 A beträgt;
der dritte vorbestimmte Stromwert 0-3,0 A beträgt; und
die zweite vorbestimmte Vorspannung 60-130 V beträgt.

13. Das Verfahren gemäß Patentanspruch 11, **dadurch gekennzeichnet, dass:**
der vierte vorbestimmte Stromwert 0,5-2,0 A beträgt;
die zweite vorbestimmte Zeitdauer 120-400 min beträgt; und
der fünfte vorbestimmte Stromwert 4,0-7,0 A beträgt.

14. Das Verfahren gemäß Patentanspruch 11, **dadurch gekennzeichnet, dass:**
der sechste vorbestimmte Stromwert 4,0-7,0 A beträgt; und
die dritte vorbestimmte Zeitdauer 15-20 min beträgt.

15. Das Verfahren gemäß Patentanspruch 11, **dadurch gekennzeichnet, dass** des Weiteren der Schritt einer Reinigung der Zerstäubungskathode vor der ersten Zerstäubungsstufe enthalten ist.

## Revendications

1. Un dispositif de revêtement à pulvérisation magnétron, comprenant :
une chambre de dépôt (100) ;
un support rotatif (102) sur une embase de la chambre de dépôt, possédant un axe central ;
un premier système de rotation pour l'entraînement du support rotatif de sorte qu'il tourne sur son axe central ;
une lunette (140) sur le support rotatif;
une cathode de pulvérisation comprenant deux premières cathode de pulvérisation (120a, 120b) situées sur une circonférence (104) concentrique avec l'axe central du support rotatif, et divisant de façon égale cette circonférence, et une deuxième cathode de pulvérisation (130) située sur ladite circonférence, et divisant de façon égale l'arc entre les deux premières cathodes de pulvérisation ;
les deux premières cathodes de pulvérisation étant réalisées avec le même élément chimique, à savoir un élément différent de la deuxième cathode de pulvérisation ;
**caractérisé en ce que** le dispositif comprend également une chicane (110) fixée sur le support rotatif, à travers son axe central, pour diviser à parts égales le support rotatif en deux zones distinctes (102-1, 102-2), la chicane possédant deux extrémités s'étendant au-delà des deux extrémités de la cathode de pulvérisation dans le sens de l'axe central du support rotatif.

2. Le dispositif de revêtement à pulvérisation magnétron selon la revendication 1, **caractérisé en ce que** les deux premières cathodes de pulvérisation (120a, 120b) sont des cibles de graphite, et la deuxième cathode de pulvérisation (130) est une cible de titane ou de tantale.

3. Le dispositif de revêtement à pulvérisation magnétron selon la revendication 1, **caractérisé en ce que** la largeur de la chicane (110) est supérieure au diamètre du support rotatif.

4. Le dispositif de revêtement à pulvérisation magnétron selon la revendication 3, **caractérisé en ce que** la chicane (110) se trouve à une distance de 2 à 10 cm de la circonférence (104) de l'emplacement de la cathode de pulvérisation (120a, 120b, 130).

5. Le dispositif de revêtement à pulvérisation magnétron selon la revendication 1, **caractérisé en ce que** la cathode de pulvérisation est rectangulaire.

6. Le dispositif de revêtement à pulvérisation magnétron selon la revendication 1, **caractérisé en ce que** la chicane (110) est fabriquée en titane, en aluminium, en acier inoxydable ou dans une combinaison de ces derniers.

7. Le dispositif de revêtement à pulvérisation magnétron selon la revendication 1, **caractérisé en ce qu'il** comprend en outre un deuxième système de rotation pour l'entraînement de la lunette (140) afin qu'elle tourne autour d'un axe central de cette dernière.

8. Le dispositif de revêtement à pulvérisation magnétron selon la revendication 7, **caractérisé en ce que** la lunette (140) est disposée sur le support rotatif (102) à travers une tige de support (160), ce même dispositif étant muni de multiples lunettes à certains intervalles.

9. Le dispositif de revêtement à pulvérisation magnétron selon la revendication 1, **caractérisé en ce que** la cathode de pulvérisation (120a, 120b, 130) est disposée sur une paroi interne de la chambre de dépôt.

10. Le dispositif de revêtement à pulvérisation magnétron selon la revendication 1, **caractérisé en ce que** la cathode de pulvérisation comprend en outre une autre deuxième cathode de pulvérisation située sur ladite circonférence (104) située en face de l'autre deuxième cathode de pulvérisation (130), de sorte que les quatre cathodes de pulvérisation divisent de façon égale ladite circonférence.

11. Une méthode de revêtement d'un substrat (150) avec une pellicule à multiples nano-couches, comprenant une première étape de pulvérisation, une deuxième étape de pulvérisation, et une troisième étape de pulvérisation, **caractérisée par** l'utilisation du dispositif de revêtement à pulvérisation magnétron selon la revendication 1 pour recouvrir le substrat, dans lequel :
(i) lors de la première étape de pulvérisation :
le courant de fonctionnement des deux premières cathodes de pulvérisation est réglé sur zéro.
le courant de fonctionnement de la deuxième cathode de pulvérisation est réglé sur une première valeur de courant prédéterminée, et sur un mode à tension constante,
la tension de polarisation de fonctionnement est maintenue sur une première tension de polarisation prédéterminée, puis
la pulvérisation d'une première durée prédéterminée à lieu, ensuite
toutes les cathodes de pulvérisation sont réglées en mode de tension constante,
la tension de polarisation de travail est maintenue à une deuxième tension de polarisation prédéterminée,
le courant de fonctionnement des deux premières cathodes de pulvérisation, à partir d'un courant de fonctionnement initial I1, est augmenté de ΔI1 à des intervalles d'un intervalle initial T1, jusqu'à ce qu'il atteigne une deuxième valeur de courant prédéterminée ;
le courant de fonctionnement de la deuxième cathode de pulvérisation, à partir de la première valeur de courant prédéterminée, diminue de ΔI2 à des intervalles d'un intervalle initial T2, jusqu'à ce qu'il atteigne une troisième valeur de courant prédéterminée ;
(ii) lors de la deuxième étape de pulvérisation :
toutes les cathodes de pulvérisation sont maintenues en mode de tension constante,
la tension de polarisation de fonctionnement est maintenue à la deuxième tension de polarisation prédéterminée,
le courant de fonctionnement de la deuxième cathode de pulvérisation est réglé et
maintenu sur une quatrième valeur de courant prédéterminée,
le courant de fonctionneùent des deux premières cathodes de pulvérisation est maintenu à la deuxième valeur de courant prédéterminée, ou est réglé et maintenu à une cinquième valeur de courant prédéterminée, puis la pulvérisation d'une deuxième durée prédéterminée est effectuée ;
(iii) lors de la troisième étape de pulvérisation :
la tension de polarisation de fonctionnement est maintenue à la deuxième tension de polarisation prédéterminée,
le courant et la tension de fonctionnement de la deuxième cathode de pulvérisation sont réglés sur zéro,
les deux premières cathodes de pulvérisation sont maintenues au courant de fonctionnement de la deuxième étape de pulvérisation, ou sont réglées et
maintenues à une sixième valeur de courant prédéterminée, et maintenues en mode de tension constante,
puis
la pulvérisation d'une troisième durée prédéterminée est effectuée ; et
(iv) la rotation uniforme du support de rotation est assurée tout au long desdites trois étapes de pulvérisation.

12. La méthode selon la revendication 11, **caractérisée en ce que :**
la première valeur de courant prédéterminée est 3,0 - 7,0 A ;
la tension du mode de tension constante est 800 V ;
la première tension de polarisation prédéterminée est 100 - 200 V ;
la première durée prédéterminée est 5 à 15 minutes ;
le courant de fonctionnement initial I1 est 0,5 - 2,0 A ;
le premier intervalle de temps T1 est 5 à 15 minutes ;
ΔI1 est 0,5 à 1,5 A ;
la deuxième valeur de courant prédéterminée est 4,0 - 7,0 A ;
le deuxième intervalle de temps T2 est 5 à 15 minutes ;
ΔI2 est 0,5 à 1,5 A ;
la troisième valeur de courant prédéterminée est 0 - 3,0 A ;
la deuxième tension de polarisation prédéterminée est 60 - 130 V.

13. La méthode selon la revendication 11, **caractérisée en ce que :**
la quatrième valeur de courant prédéterminée est 0,5 - 2,0 A ;
la deuxième durée prédéterminée est 120 à 400 minutes ; et
la cinquième valeur de courant prédéterminée est 4,0 - 7,0 A.

14. La méthode selon la revendication 11, **caractérisée en ce que :**
la sixième valeur de courant prédéterminée est 4,0 - 7,0 A ; et
la troisième durée prédéterminée est 15 à 20 minutes.

15. La méthode selon la revendication 11, **caractérisée en ce qu'une** étape de nettoyage de la cathode de pulvérisation est incluse en outre préalablement à la première étape de pulvérisation.
